# EUROPEAN PATENT APPLICATION

(11) **EP 4 373 213 A1**
(43) Date of publication of application: **22.05.2024**
(21) Application number: 22841694.7
(22) Date of filing: 10.03.2022
(51) Int. Cl.: H05B 41/24, A61L 9/20, H05B 47/16

(54) **LIGHT SOURCE DEVICE, DIELECTRIC BARRIER DISCHARGE LAMP LIGHTING CIRCUIT, AND DIELECTRIC BARRIER DISCHARGE LAMP LIGHTING METHOD**

(30) Priority: 12.07.2021 JP 2021114799
(71) Applicant: Ushio Denki Kabushiki Kaisha, Tokyo 100-8150 (JP)
(72) Inventor: SAMEJIMA,Takanori, Tokyo 100-8150 (JP); ODA,Koji, Tokyo 100-8150 (JP)
(74) Representative: Maiwald GmbH
(86) International application number: PCT/JP2022/010544
(87) International publication number: WO 2023/286344

(57) **Abstract**

A light source device that has achieved power saving and improved reliability without an increase in size in the entirety of the device, and a dielectric barrier discharge lamp lighting circuit and a dielectric barrier discharge lamp lighting method for achieving power saving and improved reliability without an increase in size of the entirety of the device are provided.

The light source device includes: a lighting circuit that includes a direct-current power source, a transformer, and a switching element, and generates electromotive force in a secondary winding of the transformer in accordance with switching of an ON state and an OFF state of the switching element; a dielectric barrier discharge lamp that is connected to the secondary winding of the transformer; and a controller that performs ON/OFF control on the switching element. The controller performs: a starting mode for repeating ON/OFF control on the switching element at a predetermined frequency at a time of starting; and a steady-state operation mode for alternately performing first control and second control after the dielectric barrier discharge lamp has been started, the first control being performed to repeat ON/OFF control on the switching element at the predetermined frequency, the second control being performed to maintain the switching element in the OFF state.

## Description

### TECHNICAL FIELD

The present invention relates to a light source device, and in particular, a light source device including a dielectric barrier discharge lamp. The present invention also relates to a dielectric barrier discharge lamp lighting circuit and a dielectric barrier discharge lamp lighting method.

### BACKGROUND ART

Conventionally, with respect to the lighting of a dielectric barrier discharge lamp, a primary control technique using a flyback type lighting circuit is known (see, for example, Patent Document 1). Furthermore, as the dielectric barrier discharge lamp lighting circuit, lighting circuits of a push-pull type, a full-bridge type, and the like in addition to a flyback type are known.

### PRIOR ART DOCUMENT

### PATENT DOCUMENTS

Patent Document 1: JP-A-H10-223384
Patent Document 2: JP-A-2020-92968

### SUMMARY OF THE INVENTION

### PROBLEMS TO BE SOLVED BY THE INVENTION

In recent years, techniques for using ultraviolet light in order to inactivate bacteria, viruses, or the like (hereinafter abbreviated to "pathogens" in some cases) have been being developed. The present applicant has also developed a technique for inactivating pathogens by using an excimer lamp, which is one type of dielectric barrier discharge lamp (see, for example, Patent Document 2 described above).

Furthermore, in recent years, due to an epidemic of a coronavirus infectious disease, or the like, as an inactivation treatment technique using the excimer lamp, treatment for inactivating pathogens by using ultraviolet light having a wavelength of 190 nm to 240 nm has been attracting attention. Ultraviolet light having a wavelength of 190 nm to 240 nm can inactivate pathogens, and has a negligible influence on humans or animals.

A known example of an excimer lamp that emits light having the wavelength range described above is an excimer lamp including a light emitting tube that is filled with noble gas and halogen gas as light-emitting gas. Known examples of such an excimer lamp include an excimer lamp that includes a light-emitting tube filled with krypton (Kr) and chlorine (CI), and has a main peak wavelength of about 222 nm, an excimer lamp that includes a light-emitting tube filled with krypton (Kr) and bromine (Br), and has a main peak wavelength of about 207 nm, and an excimer lamp that includes a light-emitting tube filled with argon (Ar) and fluorine (F), and has a main peak wavelength of about 193 nm.

In some cases, at the time of starting, the dielectric barrier discharge lamp needs the application of a voltage that is higher than a voltage to be applied during a steady-state operation, in order to generate initial discharge and start a lighting operation. Therefore, in some cases, many light source devices equipped with the dielectric barrier discharge lamp are provided with control or a mechanism to switch a voltage value to be applied to the dielectric barrier discharge lamp between a voltage value to be applied at the time of starting and a voltage value to be applied during the steady-state operation.

In particular, as described above, an excimer lamp in which light-emitting gas contains halogen gas having high electron affinity is more difficult to generate initial discharge in a light-emitting tube than an excimer lamp in which light-emitting gas does not contain halogen gas. Therefore, a larger number of light source devices including the excimer lamp in which light-emitting gas contains halogen gas employ a configuration in which a voltage that is higher than a voltage to be applied during a steady-state operation can be applied at the time of starting, in order to more reliably generate initial discharge.

However, it is difficult to achieve control to switch values of a voltage to be applied to the dielectric barrier discharge lamp by only performing ON/OFF control, and output voltage control using an analog circuit or a DC/DC converter for converting a voltage level is needed depending on a type of a lighting circuit. Therefore, a light source device including a mechanism for switching voltages requires a high-level circuit design technique in order to achieve desired characteristics relating to an output voltage, a transient characteristic, or the like.

Furthermore, an analog circuit has a possibility that a voltage to be output, or the like varies due to deterioration over time or the like. Therefore, a problem in reliability is likely to arise, and a complicated correction circuit or the like needs to be equipped in order to secure high reliability. Stated another way, the equipping of a mechanism for switching voltages to be applied to the dielectric barrier discharge lamp has resulted in an increase in size of the entirety of a light source device, an increase in a manufacturing cost, and a problem in reliability.

As described above, a light source device equipped with an excimer lamp that emits ultraviolet light having a wavelength of 190 nm to 240 nm has a negligible influence on humans or animals, and therefore the light source device has been expected to be utilized in various situations including facilities where people frequently gather, such as medical facilities, schools, or public offices, and vehicles such as automobiles, trains, buses, airplanes, or ships. Thus, it is requested that such a light source device save power, be reduced in size of the entirety of the device, and have high reliability.

In view of the problems described above, it is an object of the present invention to provide a light source device that has achieved power saving and improved reliability without an increase in size of the entirety of the device. It is also an object of the present invention to provide a dielectric barrier discharge lamp lighting circuit and a dielectric barrier discharge lamp lighting method for achieving power saving and improved reliability without an increase in size of the entirety of the device.

### MEANS FOR SOLVING THE PROBLEMS

A light source device according to the present invention includes: a lighting circuit that includes a direct-current power source, a transformer including a primary winding and a secondary winding, and at least one switching element, the lighting circuit being configured to switch supplying and stopping a current from the direct-current power source to the primary winding of the transformer in accordance with switching of an ON state and an OFF state of the at least one switching element, or change a direction of a current that flows through the primary winding, and generate electromotive force in the secondary winding of the transformer; a dielectric barrier discharge lamp that is connected to the secondary winding of the transformer; and a controller that performs ON/OFF control on the at least one switching element, and the controller performs a starting mode for repeating the ON/OFF control on the at least one switching element at a predetermined frequency to apply a predetermined voltage to the dielectric barrier discharge lamp at a time of starting, and a steady-state operation mode for alternately performing first control and second control after the dielectric barrier discharge lamp has been started, the first control being performed to repeat the ON/OFF control on the at least one switching element at the predetermined frequency to apply the predetermined voltage to the dielectric barrier discharge lamp, the second control being performed to maintain the at least one switching element in the OFF state during a period of time that is longer than a period of the ON/OFF control on the at least one switching element.

In the starting mode, ON/OFF control is performed on the switching element to generate electromotive force required to generate initial discharge in the dielectric barrier discharge lamp that is connected to the secondary winding of the transformer. Note that a magnitude of the electromotive force to be generated in the secondary winding of the transformer is set in advance by adjusting an output voltage of the direct-current power source, a winding number of each of the windings of the transformer, a time period when the switching element is maintained in the ON state, or the like in accordance with a type, a size, or the like of the dielectric barrier discharge lamp.

In the steady-state operation mode, in order to reduce power consumption per unit time, and reduce a load on the dielectric barrier discharge lamp or the lighting circuit, the first control to perform ON/OFF control on the switching element similarly to the starting mode, and the second control to maintain the switching element in the OFF state are alternately performed.

Stated another way, in both the starting mode and the steady-state operation mode, the same first control to perform ON/OFF control on the switching element is performed. However, the starting mode and the steady-state operation mode are different from each other in whether control (the second control) to maintain the switching element in the OFF state is included. In the starting mode and the first control of the steady-state operation mode, a frequency at which ON/OFF control on the switching element is repeated, and a voltage to be applied to the dielectric barrier discharge lamp are roughly the same. Note that "roughly the same" described here is used to indicate being included in the same range even if there is a minute error that is generated due to noise generated in the controller or variations in elements constituting a device.

Note that the light source device may be configured to temporarily perform the starting mode in the middle of execution of the steady-state operation mode after starting, for the purpose of, for example, prevention of fading away. Furthermore, the light source device may be configured to continue the starting mode during a predetermined time period even after the lighting of the dielectric barrier discharge lamp has been confirmed such that a lighting state becomes more reliably stable.

In the light source device according to the present invention, the configuration described above causes a reduction in an amount of power per unit time to be supplied to the dielectric barrier discharge lamp at the time of a lighting operation, in comparison with a case where the starting mode also continues to be performed after the lighting of the dielectric barrier discharge lamp, and this results in a reduction in power consumption. Furthermore, after starting, a voltage that is high enough to generate initial discharge in a light-emitting tube of the dielectric barrier discharge lamp continues to be applied, but the performing of the second control reduces the frequency of application of the voltage in comparison with the starting mode. This reduces a load on the dielectric barrier discharge lamp or the lighting circuit.

Furthermore, the steady-state operation mode for repeating the first control and the second control is achieved by switching a control signal to be input to the switching element. Stated another way, the steady-state operation mode can be achieved by not switching an analog circuit configuration to change an output voltage but performing digital signal processing to perform switching.

Moreover, in a case where the controller is constituted by a programmable device, an MCU, or the like, the light source device having the configuration described above can be achieved by rewriting a program specifying a pattern of a control signal without the need for a change in a circuit configuration, or the like. Furthermore, even in a case where the light source device having the configuration described above is achieved by a configuration of a control signal for performing ON/OFF control on the switching element at a predetermined period and a signal for masking that is output from the controller separately from the control signal of the switching element, the light source device can be achieved by using at least a single logic gate element (for example, an AND gate element for obtaining a logical product of the control signal and the signal for masking).

Stated another way, the light source device having the configuration described above does not require a complicated mechanism or circuit, and therefore the light source device can be achieved without an increase in size of the entirety of the device in comparison with a conventional device, and the number of members can be reduced depending on a configuration or the like of the lighting circuit, and the size of the entirety of the device can be reduced. Furthermore, the light source device can be achieved by only performing digital signal processing, and therefore variations in an output value or a change in characteristics due to deterioration over time does not need to be considered, and this reduces concerns about reliability.

In the light source device, the controller may be configured to periodically perform the first control and the second control in the steady-state operation mode.

In the steady-state operation mode, if the first control and the second control are repeated in a specified period, an integrated amount of irradiation of a region or an article that is irradiated with light emitted from the dielectric barrier discharge lamp becomes roughly constant in every period. Accordingly, the light source device having the configuration described above makes it easy to manage the progress of inactivation treatment in a region or an article that is irradiated with ultraviolet light, in comparison with a case where execution times of the first control and the second control are arbitrarily changed in the steady-state operation mode.

Furthermore, it is sufficient if the control described above is configured to simply repeat the first control and the second control in a predetermined period. Therefore, the control described above can be easily achieved in comparison with complicated control to change a period in accordance with an inactivation treatment condition or use environment.

In the light source device, the controller may be configured to perform the steady-state operation mode to vary the number of times of repetition of ON/OFF control on the at least one switching element in the first control before and after each time of the second control.

If a current has been periodically supplied to the transformer, periodical magnetostriction is generated, and this results in generation of minute vibration that corresponds to a frequency of the current. In a case where a frequency of the vibration falls within a human audible band (about 20 Hz to 20 kHz), an abnormal noise phenomenon called "noise generation" occurs in some cases. In the present invention, in particular, there is a possibility that a frequency of an operation to switch the first control and the second control in the steady-state operation mode will be set to a frequency within the human audible band, and this can be one of the factors of noise generation.

In view of the above, by employing the configuration described above, an operation to switch the first control and the second control in the steady-state operation mode is not performed at a specified frequency, the intensity of vibration generated in the transformer due to magnetostriction is distributed into a plurality of frequency components, and this prevents noise generation. Note that a method for not performing an operation to switch the first control and the second control in the steady-state operation mode at a specified frequency may be a method for varying the execution time of the second control before and after each time of the first control, or another method.

In the light source device, it is preferable that the controller be configured to perform the steady-state operation mode in such a way that a total of an execution time of the first control and the execution time of the second control immediately after the first control is four times or more longer than a period that corresponds to the predetermined frequency, and the execution time of the second control is 100 ms or less.

If the execution time of the second control is excessively short, there is a problem in heat generation caused by excessive power supply depending on a shape or a size of the dielectric barrier discharge lamp in some cases. In view of this, it is preferable that the execution time of the second control be four times or more longer than a period in which ON/OFF control is performed on the switching element in the first control in order to sufficiently prevent heat generation in the dielectric barrier discharge lamp. It is more preferable that the execution time of the second control be 8 times or more longer than the period.

Furthermore, if the execution time of the second control is excessively long, there is a possibility that plasma in the light-emitting tube of the dielectric barrier discharge lamp will disappear or be attenuated, and the dielectric barrier discharge lamp will be turned off. Note that, in the light source device according to the present invention, in the first control in the steady-state operation mode, a frequency at which ON/OFF control on the switching element is repeated and a voltage to be applied to the dielectric barrier discharge lamp are the same as a frequency and a voltage in the starting mode. Therefore, a voltage required to generate initial discharge in the dielectric barrier discharge lamp is applied. Stated another way, even in a case where the second control is excessively long so that the dielectric barrier discharge lamp has been turned off, there is a possibility that the dielectric barrier discharge lamp can be lit again when the controller starts the next first control.

Furthermore, the first control of the steady-state operation mode may be control to only apply a pulse-like voltage necessary and sufficient times to maintain a lighting state of the dielectric barrier discharge lamp, from the viewpoint of a reduction in power consumption or a load, or the like. However, there is a possibility that the dielectric barrier discharge lamp that has been turned off will generate initial discharge in the first control of the steady-state operation mode, but a light emitting state will not become stable by the time of a transition to the next second control, and the lighting state will fail to be maintained. Accordingly, in order to stably maintain the lighting state of the dielectric barrier discharge lamp, it is preferable that the execution time of the second control of the steady-state operation mode be 100 ms or less, it is more preferable that the execution time be 80 ms or less, and it is particularly preferable that the execution time be 10 ms or less.

In the light source device, the lighting circuit may be a flyback type circuit.

Furthermore, in the light source device, the lighting circuit may be a push-pull type circuit.

Furthermore, in the light source device, the lighting circuit may be a full-bridge type circuit.

The lighting circuit of each of the types described above includes a direct-current power source, a transformer including a primary winding, and a secondary winding that is connected to the dielectric barrier discharge lamp, and at least one switching element.

The flyback type lighting circuit is a lighting circuit configured to switch supplying and stopping a current from the direct-current power source to the primary winding of the transformer in accordance with switching of the ON state and the OFF state of the switching element, and generate electromotive force in the secondary winding of the transformer.

The flyback type lighting circuit basically only requires one switching element such as an FET, and therefore there is an economic advantage in which the flyback type lighting circuit can be configured at a low cost in comparison with the other types of lighting circuits.

The push-pull type lighting circuit and the full-bridge type lighting circuit are lighting circuits configured to change a direction of a current that flows through the primary winding of the transformer in accordance with switching of the ON state and the OFF state of the switching element, and generate electromotive force in the secondary winding of the transformer. Details of a circuit configuration and an operation of each of the lighting circuits will be described later in the item "MODE FOR CARRYING OUT THE INVENTION".

In the push-pull type lighting circuit, two switching elements are alternately switched to the ON state, and the transformers are alternately driven to operate in accordance with the switching operation described above. Therefore, there is an advantage in which power efficiency is high in comparison with the flyback type lighting circuit.

In the full-bridge type lighting circuit, four switching elements are alternately switched to the ON state, and the transformers are alternately driven to operate in accordance with the switching operation described above. Therefore, the full-bridge type lighting circuit has an advantage in which power efficiency is high in comparison with the flyback type lighting circuit. Furthermore, in the full-bridge type lighting circuit, the number of switching elements is larger than the number of switching elements in the push-pull type lighting circuit, but a positive voltage and a negative voltage of the direct-current power source are alternately applied between both terminals of the primary winding of the transformer. Therefore, the full-bridge type lighting circuit has an advantage in which power conversion efficiency is further higher than power conversion efficiency of the push-pull type lighting circuit.

In the light source device in which the lighting circuit is the flyback type circuit, the at least one switching element may include a parasitic diode.

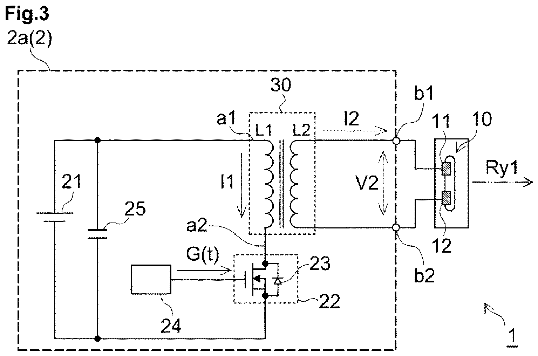

A simplest configuration in a case where the lighting circuit is the flyback type circuit is a configuration in which the direct-current power source, the primary winding of the transformer, and one switching element are connected in series (see Fig. 3). The circuit having the configuration described above performs ON/OFF control on the switching element to switch supplying and stopping a current that flows into the primary winding of the transformer (hereinafter referred to as a "primary current" in some cases), and generates electromotive force in the secondary winding.

In the flyback type lighting circuit, after the switching element has entered into the OFF state, if a current that flows through the secondary winding of the transformer (hereinafter referred to as a "secondary current" in some cases) stops, a voltage having a polarity that is reverse to a polarity of the direct-current power source is induced into the primary winding of the transformer. When this induced voltage has been generated, in a case where a circuit in which the direct-current power source, the primary winding of the transformer, and the switching element are connected in series has become an open circuit due to the switching element being in the OFF state, a high voltage is applied between input/output terminals of the switching element, and in the worst case, the switching element is damaged.

In view of this, the configuration described above forms a route through which a current flows from a negative-electrode side to a positive-electrode side of the direct-current power source when a voltage induced into the primary winding of the transformer has become greater than a voltage applied by the direct-current power source. In this case, a voltage to be applied between the input/output terminals of the switching element is the same as a forward voltage of the parasitic diode, and this prevents the switching element from being damaged. Note that a current that flows through the primary winding of the transformer via the parasitic diode is referred to as a "regenerative current" in some cases.

In the light source device in which the lighting circuit is the flyback type circuit, the controller may be configured to perform, in the first control, a first step of causing the at least one switching element to perform a transition from the ON state to the OFF state, and a second step of causing the at least one switching element to perform a transition from the OFF state to the ON state after a lapse of a predetermined OFF holding time from a point in time when a regenerative current flowing through the primary winding has reached a zero value, after the first step.

The dielectric barrier discharge lamp has an applied voltage suitable for lighting, but there are circumstances where it is practically difficult for the flyback type lighting circuit to change an applied voltage for the purpose of adjustment of irradiance. The reason for this is described below. If the flyback type lighting circuit simply changes a frequency (a switching frequency) at which the ON state and the OFF state are switched, a voltage to be applied to the dielectric barrier discharge lamp also changes in conjunction with a change in the frequency. Stated another way, in the flyback type lighting circuit, in order to adjust irradiance, a timing at which the ON state and the OFF state are switched needs to be adjusted, and a ratio of a time period during which the switching element is maintained in the ON state and a time period during which the switching element is maintained in the OFF state needs to be appropriately adjusted.

An amount of an increase in a primary current of the transformer depends on a time period after the second step is performed and then the primary current starts to increase from a zero value and before the switching element performs a transition to the OFF state again. Accordingly, by employing the configuration described above, a timing of performing the second step of causing the switching element to perform a transition from the OFF state to the ON state is later than a point in time when the regenerative current reaches a zero value. By adjusting a predetermined OFF holding time, a ratio of a time period during which the switching element is maintained in the ON state and a time period during which the switching element is maintained in the OFF state is adjusted.

As described above, the ratio of a time period during which the switching element is maintained in the ON state and a time period during which the switching element is maintained in the OFF state is adjusted, and therefore irradiance of lighting of the dielectric barrier discharge lamp is adjusted to a desired value.

Note that this effect is remarkably exhibited particularly in a case where the dielectric barrier discharge lamp is an excimer lamp that emits ultraviolet light.

For example, in a case where light-emitting gas contains KrCl, ultraviolet light having a main peak wavelength near 222 nm is emitted from the excimer lamp. Furthermore, for example, in a case where the light-emitting gas contains KrBr, ultraviolet light having a main peak wavelength near 207 nm is emitted from the excimer lamp. In contrast to ultraviolet light that is emitted from a low-pressure mercury lamp and contains a component of a wavelength of 254 nm, ultraviolet light having a main peak wavelength within a range of 190 nm to 240 nm is absorbed by the stratum corneum of the skin and does not proceed to an inside of the stratum corneum (a basal layer side), even when applied to a human body, and therefore a risk of DNA damage caused by absorption into cells is low. Therefore, the excimer lamp described above can be used for the purpose of inactivating bacteria or viruses in a space where there is a possibility of the presence of a human.

Although an influence of ultraviolet light emitted from the excimer lamp on human bodies is much lower than an influence of ultraviolet light emitted from the low-pressure mercury lamp, it is conceivable that some users wish to avoid long-time irradiation of a human body with ultraviolet light emitted from the excimer lamp at high irradiance. Furthermore, at the time of filing the present application, it is recommended that an integrated amount of irradiation of human bodies with ultraviolet rays be set within the regulation value specified by the American Conference of Governmental Industrial Hygienists (ACGIH).

A conceivable example of a method for actually installing an excimer lamp that emits such ultraviolet light is a method for installing the excimer lamp on a ceiling. In this case, in a case where the excimer lamp is installed on a relatively high ceiling, a sufficient distance to a human body is secured, and an integrated amount of irradiation with ultraviolet light decreases. However, in a case where the excimer lamp is installed on a relatively low ceiling, the distance to a human body is short, and the integrated amount of irradiation increases. If an attempt is made to operate the excimer lamp at high irradiance in an environment where the distance to a human body is short, it is necessary to repeat turning-on and turning-off in a ratio appropriate to achieve a predetermined integrated amount of irradiation (e.g., 1 0-second turning-on, 300-second turning-off). In the case of such a method, sterilization or inactivation is not performed during a turning-off time period of 300 seconds, and a risk of infection remains during the time period.

Therefore, in such a case, it is preferable that the excimer lamp be operated with the reduced irradiance of emitted ultraviolet light. The configuration described above enables a further reduction in a risk of infection by continuously irradiating a human body with ultraviolet light for a longer time without an influence on the human body, if the excimer lamp is lit at low irradiance.

Note that "inactivation" described here refers to a concept that includes killing bacteria or viruses or making infectivity or toxicity lost, and "bacteria" refer to microorganisms such as germs or fungi (mold).

Furthermore, in the light source device in which the lighting circuit is the flyback type circuit, the controller may be configured to perform, in the first control, a first step of causing the at least one switching element to perform a transition from the ON state to the OFF state, and a second step of causing the at least one switching element to perform a transition from the OFF state to the ON state after the first step and before a regenerative current flowing through the primary winding reaches a zero value or simultaneously with the regenerative current having reached the zero value.

By employing the configuration described above, at the moment when the switching element has performed a transition from the OFF state to the ON state, the regenerative current has a roughly zero value. Therefore, when the switching element has switched from the OFF state to the ON state, a large current does not suddenly flow into the switching element. Stated another way, the switching control described above causes a reduction in a load imposed on the switching element at the time of switching the ON state and the OFF state, and therefore the switching control contributes to extending the lifetime of the switching element.

Note that a method for causing the switching element to perform a transition between the ON state and the OFF state at a timing when voltages at both ends of the switching element have a roughly zero value, as described above, is referred to as "zero-voltage switching (ZVS)" in some cases.

A lighting circuit according to the present invention is a lighting circuit configured to light a dielectric barrier discharge lamp, the lighting circuit including: a direct-current power source; a transformer that includes a primary winding, and a secondary winding that is connected to the dielectric barrier discharge lamp; at least one switching element that is connected in series to the direct-current power source and the primary winding of the transformer, and switches supplying and stopping a current from the direct-current power source to the primary winding of the transformer in accordance with switching of an ON state and an OFF state, or changes a direction of a current that flows through the primary winding; and a controller that performs ON/OFF control on the at least one switching element, in which the controller performs a starting mode for repeating the ON/OFF control on the at least one switching element at a predetermined frequency to apply a predetermined voltage to the dielectric barrier discharge lamp at a time of starting, and a steady-state operation mode for alternately performing first control and second control after the dielectric barrier discharge lamp has been started, the first control being performed to repeat the ON/OFF control on the at least one switching element at the predetermined frequency to apply the predetermined voltage to the dielectric barrier discharge lamp, the second control being performed to maintain the at least one switching element in the OFF state during a period of time that is longer than a period of the ON/OFF control on the at least one switching element.

A dielectric barrier discharge lamp lighting method according to the present invention is a dielectric barrier discharge lamp lighting method using a lighting circuit, the lighting circuit including a direct-current power source; a transformer that includes a primary winding, and a secondary winding that is connected to a dielectric barrier discharge lamp; and at least one switching element, the lighting circuit being configured to switch supplying and stopping a current from the direct-current power source to the primary winding of the transformer in accordance with switching of an ON state and an OFF state of the at least one switching element, or change a direction of a current to be supplied to the primary winding, and generate electromotive force in the secondary winding of the transformer, the dielectric barrier discharge lamp lighting method including: a first step of repeating ON/OFF switching of the at least one switching element at a predetermined frequency to apply a predetermined voltage to the dielectric barrier discharge lamp at a time of starting; and a second step of alternately performing repeating the ON/OFF switching of the at least one switching element at the predetermined frequency, similarly to the first step, and maintaining the at least one switching element in the OFF state during a period of time that is longer than a period of the ON/OFF switching of the at least one switching element in the first step, in order to maintain a lighting state of the dielectric barrier discharge lamp after execution of the first step.

### EFFECT OF THE INVENTION

A light source device according to the present invention achieves power saving and improved reliability without an increase in size of the entirety of the device. Furthermore, a lighting circuit and a lighting method according to the present invention can achieve power saving and improved reliability without an increase in size of the entirety of the light source device.

### BRIEF DESCRIPTION OF THE DRAWINGS

Fig. 1 is a perspective view schematically illustrating an example of the appearance of a light source device.
Fig. 2 is a perspective view schematically illustrating an example of the appearance of the light source device illustrated in Fig. 1 from which some elements have been removed.
Fig. 3 is a circuit diagram illustrating a configuration example of a flyback type lighting circuit for a dielectric barrier discharge lamp.
Fig. 4 is a timing chart schematically illustrating temporal changes in a control signal G(t) and a secondary voltage V2 of the flyback type lighting circuit.
Fig. 5 is a timing chart schematically illustrating temporal changes in the control signal G(t), a primary current 11, the secondary voltage V2, and a secondary current I2 during a time period when a controller is performing ON/OFF control on a switching element.
Fig. 6 is a circuit diagram illustrating a configuration example of a push-pull type lighting circuit for the dielectric barrier discharge lamp.
Fig. 7 is a timing chart schematically illustrating temporal changes in the control signal G(t) and the secondary voltage V2 of the push-pull type lighting circuit.
Fig. 8 is a circuit diagram illustrating a configuration example of a full-bridge type lighting circuit for the dielectric barrier discharge lamp.
Fig. 9 is a timing chart schematically illustrating temporal changes in the control signal G(t) and the secondary voltage V2 of the flyback type lighting circuit.
Fig. 10 is a timing chart schematically illustrating temporal changes in the control signal G(t), the primary current 11, the secondary voltage V2, and the secondary current I2 during a time period when the controller is performing ON/OFF control on a switching element 22.

### MODE FOR CARRYING OUT THE INVENTION

A light source device, a dielectric barrier discharge lamp lighting circuit, and a dielectric barrier discharge lamp lighting method according to the present invention will be described below with reference to the drawings. Note that the respective drawings described below regarding the light source device are all schematic illustrations, and the dimensional ratios and numbers of parts on the drawings do not necessarily match the actual dimensional ratios and numbers of parts.

### [Configuration]

### (Light source device 1)

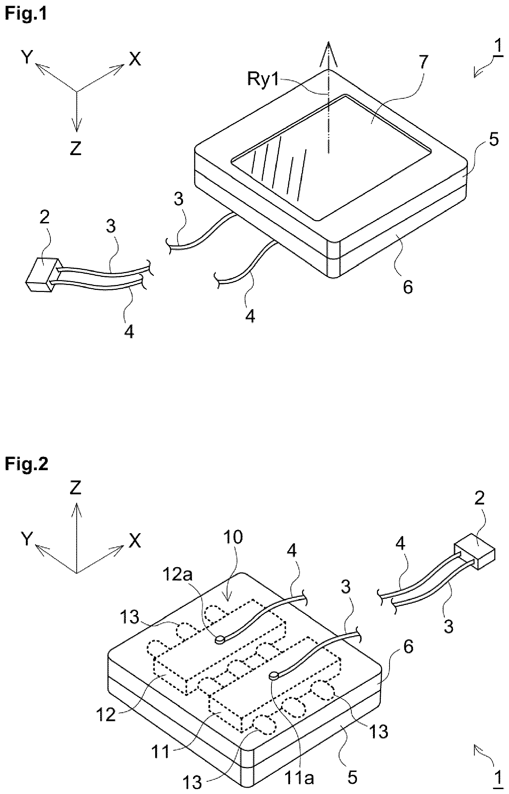

Figs. 1 and 2 are perspective views schematically illustrating the appearance of a light source device 1. However, the structure illustrated in Figs. 1 and 2 is merely an example, and the light source device 1 according to the present invention has an arbitrary structure.

Each of Figs. 1 and 2 is a perspective view schematically illustrating an example of the appearance of the light source device 1. In Fig. 2, some elements have been removed from Fig. 1 for the sake of description. As illustrated in Fig. 1, the light source device 1 includes a lighting circuit 2, a cover 5 having a light extraction surface 7 formed on one surface, and a main body casing 6. In the example illustrated in Fig. 2, the light source device 1 includes a dielectric barrier discharge lamp 10 that is constituted by a plurality of light-emitting tubes 13 and electrodes (11, 12) for applying a voltage to each of the light-emitting tubes 13. The electrodes (11, 12) are respectively connected to power lines (3, 4) by using connecting parts (11a, 12a). The power lines (3, 4) are connected to the lighting circuit 2.

The light-emitting tubes 13 are made of a dielectric material such as quartz glass, and are filled with predetermined light-emitting gas. If a high-frequency voltage of, for example, about 1 kHz to 5 MHz is applied to the electrodes (11, 12), the voltage is applied to the light-emitting gas via the light-emitting tubes 13. In this case, discharge plasma is generated in a discharge space filled with the light-emitting gas, atoms of the light-emitting gas are excited to an excimer state, and excimer light emission occurs when the atoms shift to a ground state. Light that has been emitted from the dielectric barrier discharge lamp 10 due to this excimer light emission is emitted as light Ry1 from the light extraction surface 7 to an outside of the light source device 1.

A wavelength of the light Ry1 emitted from the light source device 1 is determined dependently on a substance contained in the light-emitting gas that the light-emitting tubes 13 are filled with. For example, in a case where the light-emitting gas contains KrCl, the light Ry1 emitted from the light source device 1 indicates a spectrum having a main peak wavelength near 222 nm. In a case where the light-emitting gas contains KrBr, the light Ry1 indicates a spectrum having a main peak wavelength near 207 nm. In a case where the light-emitting gas contains ArF, the light Ry1 indicates a spectrum having a main peak wavelength near 193 nm.

However, in the present invention, the type of gas that the light-emitting tubes 13 of the dielectric barrier discharge lamp 10 are filled with is arbitrary, and may be appropriately selected depending on a desired wavelength of the light Ry1. Furthermore, in the light source device 1, for the purpose of shifting the wavelength to a longer wavelength side, a fluorescent material may be applied onto tube walls of the light-emitting tubes 13 or the light extraction surface 7.

Furthermore, the tube walls of the light-emitting tubes 13 or the light extraction surface 7 may include a filter that transmits ultraviolet light having a wavelength band that has a small effect on human bodies, and does not transmit ultraviolet light having a wavelength band that has a significant effect on human bodies. As the filter, for example, a dielectric multilayer film filter configured to transmit ultraviolet light having a wavelength of 190 nm to 240 nm and not transmit ultraviolet light having a wavelength of 240 nm or more, or the like can be employed.

### (Flyback type lighting circuit 2a)

Fig. 3 is a circuit diagram illustrating a configuration example of a flyback type lighting circuit 2a for a dielectric barrier discharge lamp. The flyback type lighting circuit 2a is an example of the lighting circuit 2 that is configured to light the dielectric barrier discharge lamp 10 and includes a direct-current power source 21, a single switching element 22, and a transformer 30, as illustrated in Fig. 3.

The transformer 30 includes a primary winding L1 and a secondary winding L2. From among terminals included in the primary winding L1 of the transformer 30, a first terminal a1 is connected to a positive-electrode side terminal of the direct-current power source 21, and a second terminal a2 is connected to a negative-electrode side terminal of the direct-current power source 21 via the switching element 22.

The switching element 22 according to the present embodiment is constituted by a field effect transistor (FET), and includes a parasitic diode 23 in which an anode is connected to the negative-electrode side terminal of the direct-current power source 21 and a cathode is connected to the primary winding L1 of the transformer 30. In the present embodiment, this parasitic diode 23 functions as a regenerative circuit. Note that as the switching element 22, an element as opposed to the field effect transistor (FET) may be employed. Furthermore, as the switching element 22, an IGBT, a relay element, or the like that does not include the parasitic diode 23 may be employed, and a single diode element may be connected in parallel to the switching element 22 to form the regenerative circuit.

The direct-current power source 21 may be constituted by, for example, an AC/DC converter that performs AC/DC conversion on a not-illustrated commercial power supply. The lighting circuit 2a includes a smoothing capacitor 25 to smoothen a voltage waveform. The direct-current power source 21 may be constituted by a battery.

The lighting circuit 2a according to the present embodiment includes a controller 24 configured to perform ON/OFF control on the switching element 22. It is sufficient if the controller 24 outputs a control signal G(t) having a desired pattern, and, for example, a CPU, an MPU, or the like, which is a control unit, can be employed. Hereinafter, the content of control performed by the controller 24 will be described with reference to a timing chart as well.

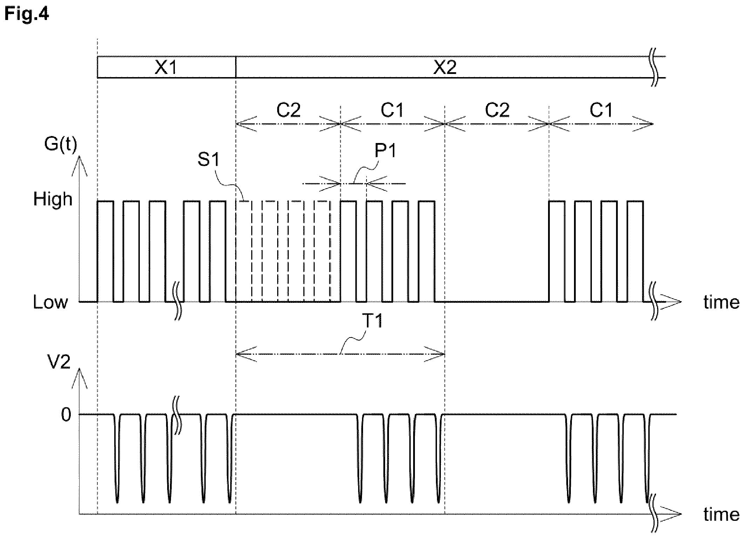

Fig. 4 is a timing chart schematically illustrating temporal changes in a control signal G(t) and a secondary voltage V2 of the flyback type lighting circuit 2a. The timing chart illustrated in Fig. 4 indicates changes in the control signal G(t) and the secondary voltage V2 after the dielectric barrier discharge lamp 10 has started. Furthermore, in a graph indicating the control signal G(t) in Fig. 4, a High level (hereinafter referred to as an "H level") corresponds to ON control performed on the switching element 22, and a Low level (hereinafter referred to as an "L level") corresponds to OFF control performed on the switching element 22. Moreover, a graph indicating the secondary voltage V2 in Fig. 4 indicates a potential difference from a first electrode terminal b1 connected to the electrode 11 at the time of using, as a reference, a potential of a second electrode terminal b2 connected to the electrode 12 of the dielectric barrier discharge lamp 10 illustrated in Fig. 3.

Graphs indicating a change in a voltage or a current illustrated in the drawings referred to in the description below do not indicate an offset or the like that scarcely affects the description of a principal operation of the present invention, and schematically indicate an example of an ideal waveform, similarly to the graph indicating the secondary voltage V2 in Fig. 4. Furthermore, whether the secondary voltage V2 shifts to a + side or a - side relative to a predetermined reference voltage (in the present embodiment, 0 V) may be appropriately and arbitrarily set according to the specifications of the dielectric barrier discharge lamp 10, a configuration of the lighting circuit 2, or the like.

When the light source device 1 has been turned on to start an operation, the controller 24 switches to a starting mode X1 for lighting the dielectric barrier discharge lamp 10.

In the starting mode X1, control to repeat ON/OFF control on the switching element 22 at a predetermined frequency is continuously performed, as illustrated in Fig. 4. The starting mode X1 is performed after discharge plasma has occurred in the light-emitting tubes 13 of the dielectric barrier discharge lamp 10 and before a light emitting state of the dielectric barrier discharge lamp 10 becomes stable.

After the controller 24 has performed the starting mode X1, and the light emitting state of the dielectric barrier discharge lamp 10 has becomes stable, and stated another way, after the dielectric barrier discharge lamp 10 has started, the controller 24 switches to a steady-state operation mode X2 for maintaining a lighting state of the dielectric barrier discharge lamp 10.

In the steady-state operation mode X2, as illustrated in Fig. 4, first control C1 to repeat ON/OFF control on the switching element 22 at a predetermined frequency to apply a predetermined voltage and second control C2 to maintain the switching element 22 in an OFF state are alternately repeated, and the lighting state of the dielectric barrier discharge lamp 10 is maintained. Note that a frequency at which ON/OFF control on the switching element 22 is repeated and a predetermined voltage to be applied to the dielectric barrier discharge lamp 10 in the first control C1 are roughly the same as a frequency at which ON/OFF control on the switching element 22 is repeated and a predetermined voltage to be applied to the dielectric barrier discharge lamp 10 in the starting mode X1.

In the steady-state operation mode X2 according to the present embodiment, the switching element 22 is controlled, by using a control signal G(t) obtained by performing mask processing on a basic control signal S1 for switching the H/L levels at a predetermined frequency in such a way that an output of the basic control signal S1 is only fixed to the L level during a period of time when the second control C2 is performed (in Fig. 4, a portion of mask processing is illustrated with a broken line). Therefore, as illustrated in Fig. 4, the first control C1 in the steady-state operation mode X2 is only different in an execution time from the starting mode X1, and in both cases, the secondary voltage V2 having the same pattern is applied between the electrodes (11, 12) of the dielectric barrier discharge lamp 10, as illustrated in Fig. 3.

Examples of the mask processing described here include processing for periodically fixing the control signal G(t) to the L level during a predetermined period of time, and processing for inputting, to an AND gate element, the basic control signal S1 and a signal for masking that causes an output of the basic control signal S1 to be fixed to the H level in a section where the first control C1 is performed and be fixed to the L level in a section where the second control C2 is performed to obtain a logical product, by performing processing on a program.

In the present embodiment, it has been set that a period P1 of the basic control signal S1 for repeating ON/OFF control on the switching element 22 is 17 µs, an execution time of the starting mode X1 is 10 s, an execution time of the first control C1 in the steady-state operation mode X2 is 170 µs, and an execution time for the second control C2 of the steady-state operation mode X2 is 200 µs. Stated another way, the execution time of the second control C2 of the steady-state operation mode X2 is 12 times longer than the period P1 of the basic control signal S1, and a period T1 of repeating the first control C1 and the second control C2 in the steady-state operation mode X2 is 22 times longer than the period P1 of a basic signal frequency.

It is preferable that the period T1 of repeating the first control C1 and the second control C2 in the steady-state operation mode X2 be four times or more longer than the period P1 of the basic control signal S1 in order to prevent heat generation in the dielectric barrier discharge lamp 10, and it is more preferable that the period T1 be 8 times or more longer than the period P1. Note that in the steady-state operation mode X2, in a case where the first control C1 and the second control C2 are aperiodically repeated, it is preferable that the total of the execution times of the first control C1 and the previous second control C2 be four times or more longer than the period P1 of the basic control signal S1, and it is more preferable that the total be 8 times or more longer than the period P1.

Furthermore, if the execution time of the second control C2 is excessively long, there is a possibility that plasma in the light-emitting tubes 13 of the dielectric barrier discharge lamp 10 will disappear or be attenuated, and the dielectric barrier discharge lamp 10 will be turned off. As described above, in the first control C1 of the steady-state operation mode X2, the same voltage is applied to the dielectric barrier discharge lamp 10 in the same period as a period of the starting mode X1, and therefore there is a possibility that the dielectric barrier discharge lamp 10 that has been turned off will be turned on again. However, there is a possibility that a voltage will continue to be applied during an insufficient time period, a light emitting state will be unstable before a shift to the next second control C2, and the lighting state will fail to be maintained. Accordingly, in order to stably maintain the lighting state of the dielectric barrier discharge lamp 10, it is preferable that the execution time of the second control C2 of the steady-state operation mode X2 be 100 ms or less, and it is more preferable that the execution time be 80 ms or less. Moreover, in order to further improve stability of the lighting state, it is preferable that the execution time of the second control C2 of the steady-state operation mode X2 be 10 ms or less, and it is more preferable that the execution time be 1 ms or less.

Here, an intensity of light emitted from the dielectric barrier discharge lamp 10 is described. The intensity of the light emitted from the dielectric barrier discharge lamp 10 is adjusted according to a ratio of the execution time of the first control C1 and the execution time of the second control C2 per unit time. Specifically, in the period T1 in the steady-state operation mode X2, if a ratio of the execution time of the first control C1 increases, an intensity of the light emitted from the dielectric barrier discharge lamp 10 increases. In contrast, if a ratio of the execution time of the second control C2 increases, an intensity of the light emitted from the dielectric barrier discharge lamp 10 decreases.

An example is described below for reference. When it is assumed that an intensity of light near the light-emitting tubes 13 of the dielectric barrier discharge lamp 10 in the starting mode X1 is 100%, an intensity of light near the light-emitting tubes 13 of the dielectric barrier discharge lamp 10 in the steady-state operation mode X2 in which the execution time of the first control C1 has been adjusted to 1 ms, and the execution time of the second control C2 has been adjusted to 100 µs is about 91%. Furthermore, an intensity of light near the light-emitting tubes 13 of the dielectric barrier discharge lamp 10 in the steady-state operation mode X2 in which the execution time of the first control C1 has been adjusted to 1 ms, and the execution time of the second control C2 has been adjusted to 1 ms is about 50%.

Accordingly, the light source device 1 can be configured to emit light having a desired intensity depending on the place of use or the purpose of use, by adjusting the execution time of the first control C1 and the execution time of the second control C2 in the period T1 in the steady-state operation mode X2. Note that the execution time of the first control C1 and the execution time of the second control C2 in the steady-state operation mode X2 may be adjusted and fixed in advance, may be variable according to the purpose of use or a time zone of operation, or may always vary.

Furthermore, as a method for adjusting an intensity of light emitted from the dielectric barrier discharge lamp 10, a method for causing the first control C1 to vary an execution time in the steady-state operation mode X2 may be employed. As a specific example, a mode for fixing the execution time of the second control C2 to 1 ms, and alternately switching the execution time of the first control C1 between 1 ms and 0.5 ms every predetermined times or every predetermined time periods may be employed.

Moreover, the controller 24 may be configured to temporarily switch to the starting mode X1 in the middle of execution of the steady-state operation mode X2 for the purpose of, for example, prevention of fading away. Furthermore, the controller 24 may be configured to continue the starting mode X1 during a predetermined time period even after the lighting of the dielectric barrier discharge lamp 10 has been confirmed in order to make the lighting state more reliably stable.

Furthermore, in the present embodiment, an ON time and an OFF time of the basic control signal S1 have been adjusted in such a way that the secondary voltage V2 has a peak voltage of 5 to 6 kV. The peak voltage of the secondary voltage V2 is appropriately adjusted according to a shape or a size of the dielectric barrier discharge lamp 10 or light-emitting gas that the light-emitting tubes 13 are filled with.

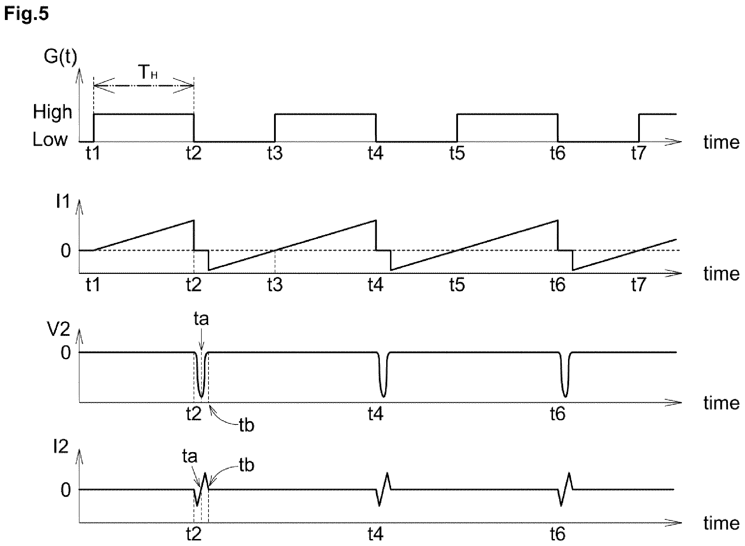

Next, details of operations of the starting mode X1 and the first control C1 of the steady-state operation mode X2 are described with reference to the configuration illustrated in Fig. 3 of the lighting circuit 2a and a timing chart. Fig. 5 is a timing chart schematically illustrating temporal changes in the control signal G(t), a primary current 11, the secondary voltage V2, and a secondary current I2 during a time period when the controller 24 is performing ON/OFF control on the switching element 22.

As illustrated in Fig. 5, when the control signal G(t) has changed from the L level to the H level at time t1, the switching element 22 performs a transition from an OFF state to an ON state, and the primary current I1 of the transformer 30 increases as time passes.

Then, when the control signal G(t) has changed from the H level to the L level at time t2, the switching element 22 performs a transition from the ON state to the OFF state. At this time, back electromotive force is generated in the secondary winding L2 of the transformer 30, and a pulse-like secondary voltage V2 is generated. When this secondary voltage V2 has been applied to an inside of the light-emitting tubes 13 of the dielectric barrier discharge lamp 10 via the pair of electrodes (11, 12), light Ry1 is emitted from the dielectric barrier discharge lamp 10.

Note that a magnitude of the secondary voltage V2 that is generated in the secondary winding L2 of the transformer 30 depends on a change amount of the primary current I1 at time t2. In addition, the change amount of the primary current I1 at time t2 depends on a time period (a time period T_{H}) during which the H level of the control signal G(t) is maintained. Therefore, the time period T_{H} of the control signal G(t) according to the present embodiment has been set to a time period that causes the secondary voltage V2 to have a voltage value required to generate initial discharge in the light-emitting tubes 13 of the dielectric barrier discharge lamp 10.

The application of the secondary voltage V2 causes the secondary current I2 to flow through the secondary winding L2 of the transformer 30. The secondary current I2 flows while releasing energy stored in the transformer 30, and therefore the secondary current I2 becomes closer to a zero value as time passes (time t2 to time ta).

In this case, the dielectric barrier discharge lamp 10 includes the light-emitting tubes 13 made of a dielectric material, and includes the pair of electrodes (11, 12) to clamp the light-emitting tube 13, and the dielectric barrier discharge lamp 10 can be regarded as a capacitor element that equivalently stores electric charge. Stated another way, the release of energy stored in the transformer 30 causes electric charge to be gradually stored in the dielectric barrier discharge lamp 10.

When the secondary current I2 has completed the release of energy stored in the transformer 30, the electric charge stored in the dielectric barrier discharge lamp 10 is discharged. This discharge causes a current (the secondary current I2) to flow through the secondary winding L2 of the transformer 30 in a direction reverse to the previous direction, and the secondary voltage V2 changes to be closer to a zero value (time ta to time tb).

After the completion of discharge of the dielectric barrier discharge lamp 10, similarly, the secondary winding L2 of the transformer 30 serves as a voltage source, and continuously causes the secondary current I2 to flow while electrically charging the dielectric barrier discharge lamp 10. Then, when the flow of the secondary current I2 has stopped, a primary voltage V1 is induced in the primary winding L1 of the transformer 30.

This induced voltage is a voltage having a polarity reverse to a polarity of the direct-current power source 21. However, as described above, the switching element 22 includes the parasitic diode 23, and therefore the primary current I1 flows through the primary winding L1 in a reverse direction via the parasitic diode 23. This primary current I1 is referred to as a "regenerative current" in some cases. Such a regenerative current is generated as a result of circumstances specific to a load constituted by the dielectric barrier discharge lamp 10.

The primary current I1 gradually becomes closer to a zero value. However, when the switching element 22 has entered into the ON state again at time t3, a value of the primary current I1 continues to increase similarly to time t1 to time t2. After this, similar control is repeatedly performed.

In the present embodiment, at the same time as the primary current I1 has reached the zero value, control is performed to cause the switching element 22 to perform a transition from the OFF state to the ON state (time t3). Stated another way, zero-voltage switching is performed. Note that in consideration of transmission delay of the control signal G(t) or a time period required for the switching element 22 to switch from the ON state to the OFF state, control to cause the switching element 22 to perform a transition from the OFF state to the ON state may be performed before the primary current I1 reaches the zero value.

Control to cause the switching element 22 to perform a transition from the ON state to the OFF state (e.g., control at times t2, t4, and t6 in Fig. 5) corresponds to the "first step", and control to cause the switching element 22 to perform a transition from the OFF state to the ON state (e.g., control at times t1, t3, t5, and t7 in Fig. 5) corresponds to the "second step".

The light source device 1 having the configuration described above can be achieved by performing digital signal processing to perform switching without the need for switching an analog circuit configuration to change an output voltage. Accordingly, a complicated mechanism or circuit is not required, and therefore a size of the entirety of the device does not increase in comparison with a conventional device, and in some cases, the number of members can be reduced, and the size of the entirety of the device can be reduced.

Furthermore, the light source device 1 having the configuration described above can be achieved by only performing digital signal processing, and therefore variations in an output value or analog characteristics such as a change in characteristics due to deterioration over time do not need to be considered, and a problem in reliability is not likely to occur.

Hereinafter, configuration examples of lighting circuits 2 of types as opposed to the flyback type, and primarily operations of the starting mode X1 and the first control C1 of the steady-state operation mode X2 performed by the respective lighting circuits 2 are described.

### (Push-pull type lighting circuit 2b)

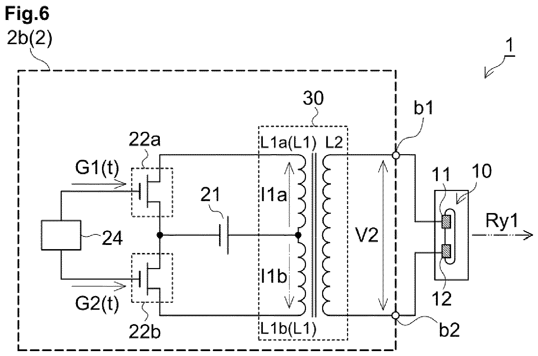

Fig. 6 is a circuit diagram illustrating a configuration example of a push-pull type lighting circuit 2b for the dielectric barrier discharge lamp. The push-pull type lighting circuit 2b is an example of the lighting circuit 2 that is configured to light the dielectric barrier discharge lamp 10 and includes the direct-current power source 21, two switching elements (22a, 22b), and the transformer 30, as illustrated in Fig. 6. On a primary side of the transformer 30, a circuit in which a winding L1a and a switching element 22a are connected in series and a circuit in which a winding L1b and a switching element 22b are connected in series are connected in parallel to the direct-current power source 21.

In the transformer 30 according to the present embodiment, the winding L1a and the winding L1b constitute the primary winding L1 of the transformer 30. Note that in the transformer 30 in the present configuration, the primary winding L1 has been divided into two windings (L1a, L1b) for convenience of description. However, the transformer 30 may include a single primary winding L1.

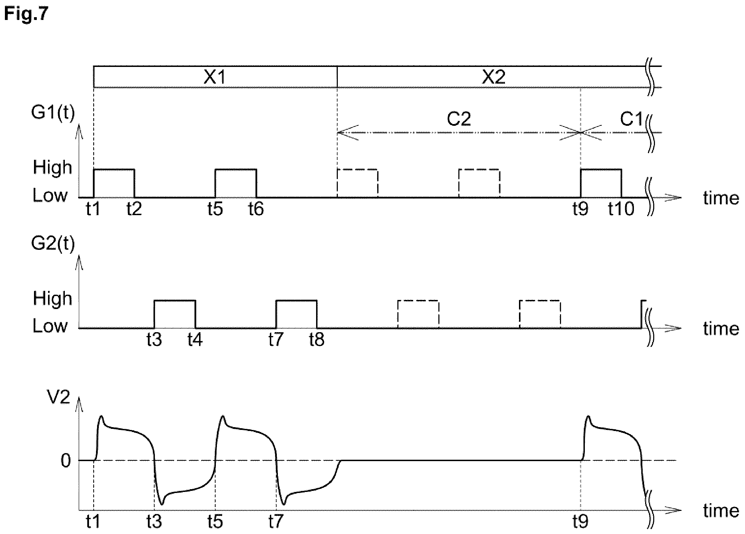

Fig. 7 is a timing chart schematically illustrating temporal changes in a control signal G1(t), a control signal G2(t), and the secondary voltage V2 of the push-pull type lighting circuit 2b. In the push-pull type lighting circuit 2b, in the starting mode X1 and the first control C1 of and the steady-state operation mode X2, the controller24 performs control to output the control signal G1(t) to the switching element 22a and output the control signal G2(t) to the switching element 22b, and alternately switch the respective switching elements (22a, 22b) to the ON state. Hereinafter, an operation of the starting mode X1 or the first control C1 of the steady-state operation mode X2 in the push-pull type lighting circuit 2b is described with reference to Figs. 6 and 7.

When the starting mode X1 or the first control C1 of the steady-state operation mode X2 has been started, the controller 24 included in the push-pull type lighting circuit 2b switches the control signal G1(t) from the L level to the H level, and switches the switching element 22a to the ON state (time t1). When the switching element 22a has switched to the ON state, the current I1a starts to flow from the direct-current power source 21 to a side of the winding L1a. When the current I1a has started to flow through the winding L1a, the secondary voltage V2 illustrated in Fig. 7 is generated in the secondary winding L2 of the transformer 30.

Next, the controller 24 switches the control signal G1(t) from the H level to the L level to switch the switching element 22a to the OFF state (time t2), and then switches the control signal G2(t) from the L level to the H level to switch the switching element 22b to the ON state (time t3). When the switching element 22b has switched to the ON state, a current I1b starts to flow from the direct-current power source 21 to a side of the winding L1b. When the current I1b has started to flow through the winding L1b, the secondary voltage V2 illustrated in Fig. 7 is generated in the secondary winding L2 of the transformer 30.

A flowing direction of the current I1b generated in the primary winding L1 of the transformer 30 in an operation at time t3 is reverse to a flowing direction of the current I1a generated in the primary winding L1 in an operation at time t1. Therefore, the polarity of a secondary voltage V2 that is generated in the secondary winding L2 of the transformer 30 in the operation at time t3 is reverse to the polarity of the secondary voltage V2 that is generated in the operation at time t1, as illustrated in Fig. 7.

Then, the controller 24 outputs a control signal G2(t) for switching the switching element 22b to the OFF state (time t4). After this, until control is switched to the steady-state operation mode X2 or until control is switched to the second control C2, similar control is repeatedly performed.

Note that it is preferable that control to switch the switching element 22b to the ON state (time t3) be performed slightly after control to switch the switching element 22a to the OFF state (time t2). The reason for this is to avoid a situation where a period of time during which the two switching elements (22a, 22b) simultaneously enter into the ON state is generated, and this results in short-circuit between the positive-electrode side terminal and the negative-electrode side terminal of the direct-current power source 21.

### (Full-bridge type lighting circuit 2c)

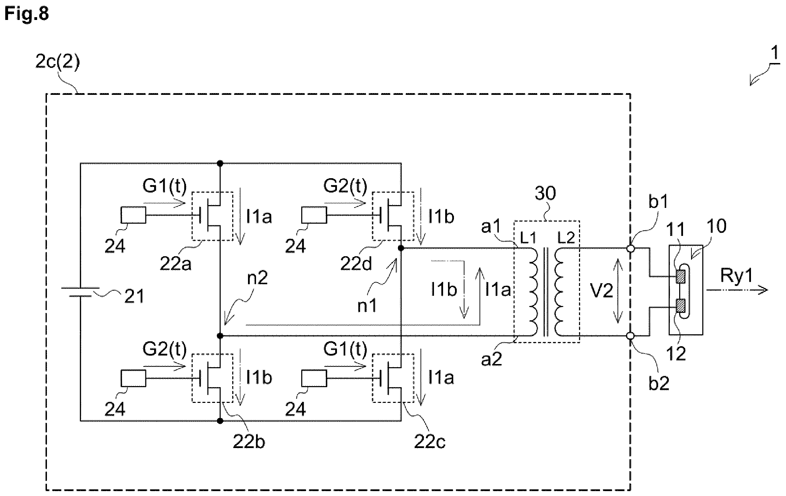

Fig. 8 is a circuit diagram illustrating a configuration example of a full-bridge type lighting circuit 2c for the dielectric barrier discharge lamp. The full-bridge type lighting circuit 2c is an example of the lighting circuit 2 that is configured to light the dielectric barrier discharge lamp 10 and includes the direct-current power source 21, four switching elements (22a, 22b, 22c, 22d), and the transformer 30, as illustrated in Fig. 8. A primary side of the transformer 30 has a circuit configuration in which a circuit in which two switching elements (22a, 22b) are connected in series and a circuit in which two switching elements (22c, 22d) are connected in series are connected in parallel to the direct-current power source 21. The primary winding L1 of the transformer 30 is connected to a first node n1 between the switching elements (22a, 22b), and a second node n2 between the switching elements (22c, 22d). In Fig. 8, for convenience of illustration, the controller 24 has been divided into four pieces, but a single controller 24 may be employed similarly to the lighting circuits (2a, 2b) of the other types.

The full-bridge type lighting circuit 2c is different from the push-pull type lighting circuit 2b in a circuit configuration and a magnitude of the secondary voltage V2 that is generated in the secondary winding L2 of the transformer 30. However, the full-bridge type lighting circuit 2c is the same as the push-pull type lighting circuit 2b in that ON/OFF control is performed on pairs of switching elements (22a, 22b, 22c, 22d) by using two control signals (G1(t), G2(t)) to change directions of currents (I1a, I1b) that flow through the primary winding L1 and alternately generate electromotive force having a reverse polarity in the secondary voltage V2. Therefore, a timing chart of the control signals (G1(t), G2(t)) and the secondary voltage V2 that are illustrated in Fig. 8 is almost the same as the timing chart illustrated in Fig. 7 of the push-pull type lighting circuit 2b, excluding a difference in a scale of the secondary voltage V2, or the like. Hereinafter, an operation of the starting mode X1 or the first control C1 of the steady-state operation mode X2 in the full-bridge type lighting circuit 2c is described with reference to Figs. 7 and 8.

When the starting mode X1 or the first control C1 of the steady-state operation mode X2 has been started, the controller 24 included in the full-bridge type lighting circuit 2c switches the control signal G1(t) from the L level to the H level, and switches the switching elements (22a, 22c) to the ON state (time t1). When the switching elements (22a, 22c) have switched to the ON state, the current I1a starts to flow from the direct-current power source 21 through a route of the switching element 22a, the primary winding L1, and the switching element 22c. When the current I1a has started to flow through the primary winding L1, the secondary voltage V2 is generated.

Next, the controller 24 switches the control signal G1(t) from the H level to the L level to switch the switching elements (22a, 22c) to the OFF state (time t2), and then switches the control signal G2(t) from the L level to the H level to switch the switching elements (22b, 22d) to the ON state (time t3). When the switching elements (22b, 22d) have switched to the ON state, the current I1b starts to flow from the direct-current power source 21 through a route of the switching element 22d, the primary winding L1, and the switching element 22b. When the current I1b has started to flow through the primary winding L1, the secondary voltage V2 is generated.

A flowing direction of the current I1b generated in the primary winding L1 of the transformer 30 in an operation at time t3 is reverse to a flowing direction of the current I1a generated in the primary winding L1 in an operation at time t1. Therefore, the polarity of a secondary voltage V2 that is generated in the secondary winding L2 of the transformer 30 in the operation at time t3 is reverse to the polarity of the secondary voltage V2 that is generated in the operation at time t1, as illustrated in Fig. 7.

Then, the controller 24 switches the control signal G2(t) from the H level to the L level in order to switch the switching elements (22b, 22d) to the OFF state (time t4). After this, until control is switched to the steady-state operation mode X2 or until control is switched to the second control C2, similar control is repeatedly performed.

Note that it is preferable that control to switch the switching elements (22b, 22d) to the ON state (time t3) be performed slightly after control to switch the switching elements (22a, 22c) to the OFF state (time t2). Similarly to the push-pull type lighting circuit 2b, the reason for this is to avoid a situation where a period of time during which the switching elements (22a, 22b, 22c, 22d) simultaneously enter into the ON state is generated, and this results in short-circuit between the positive-electrode side terminal and the negative-electrode side terminal of the direct-current power source 21.

Three types of lighting circuits (2a, 2b, 2c) have been described above, but the lighting circuit 2 of a type as opposed to the types of the lighting circuits (2a, 2b, 2c) described above, such as a half-bridge type, may be employed.

### [Other embodiments]

Hereinafter, other embodiments of the light source device 1 or the lighting circuit 2 according to the present invention will be described.

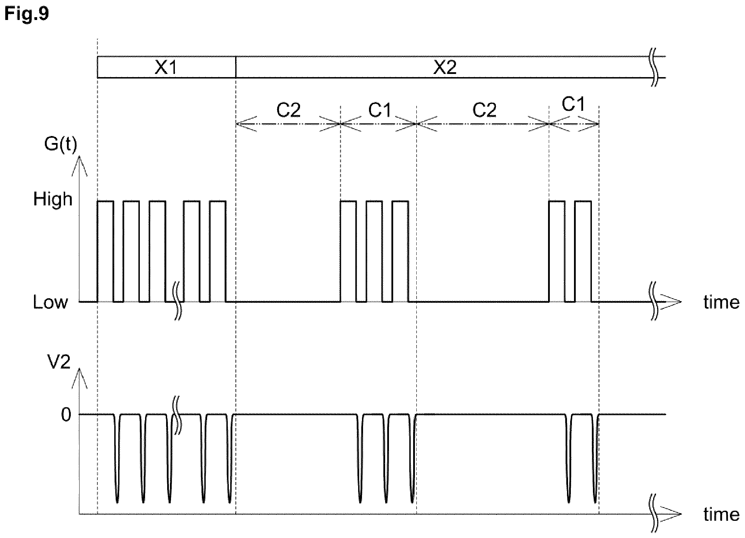

<1> Fig. 9 is a timing chart schematically illustrating temporal changes in the control signal G(t) and the secondary voltage V2 in the flyback type lighting circuit 2a (see Fig. 3), and the timing chart of Fig. 9 is different from the timing chart of Fig. 4. The controller 24 according to the present embodiment performs control in such a way that, in the first control C1 of the steady-state operation mode X2, the number of times of repetition of ON/OFF control on the switching element 22 varies before and after the second control C2. For example, as illustrated in Fig. 9, in the first control C1 that is performed first after switching to the steady-state operation mode X2, ON/OFF control on the switching element 22 is repeated three times, and in the first control C1 that follows, ON/OFF control on the switching element 22 is repeated two times. In the present embodiment, after this, the first control C1 is performed in such a way that the number of times of repetition of ON/OFF control on the switching element 22 changes in such a way that three times => two times => three times => two times.... Note that in the first control C1, the number of times of repetition of ON/OFF control on the switching element 22 may change at random in contrast to periodical repetition of some patterns.

As described above, when a periodical current has been supplied to the transformer, periodical magnetostriction is generated, and this results in generation of minute vibration that corresponds to a frequency of the current. In a case where a frequency of the vibration falls within a human audible band (about 20 Hz to 20 kHz), noise generation occurs in some cases.

In view of this, as described above, the number of times of repetition of ON/OFF control on the switching element 22 in the first control C1 is changed before and after each second control C2 in the steady-state operation mode X2, and therefore an operation to switch the first control C1 and the second control C2 is not performed at a specified frequency. Accordingly, the intensity of vibration generated in the transformer due to magnetostriction caused by an operation to switch the first control C1 and the second control C2 is distributed into a plurality of frequency components, and this prevents sound generation.

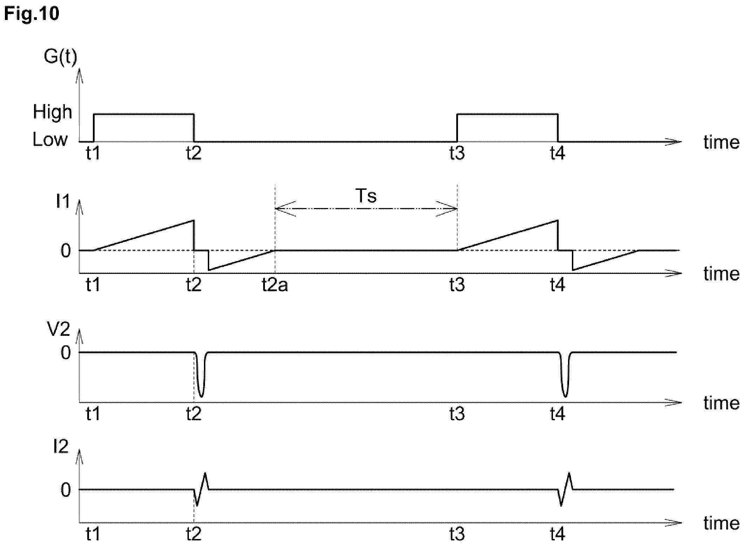

<2> Fig. 10 is a timing chart schematically illustrating temporal changes in the control signal G(t), the primary current I1, the secondary voltage V2, and the secondary current I2 during a time period when the controller 24 is performing ON/OFF control on the switching element 22, and the timing chart of Fig. 10 is different from the timing chart of Fig. 5. In the flyback type lighting circuit 2a according to the present embodiment (see Fig. 3), an OFF holding time Ts is intentionally provided, as illustrated in Fig. 10, and therefore a timing of causing the switching element 22 to perform a transition from the OFF state to the ON state is delayed.

More specifically, as illustrated in Fig. 10, at time t3 after time t2a when the primary current I1 during a regenerative operation (a regenerative current) has reached a zero value, the controller 24 changes the control signal G(t) from the L level to the H level, and the switching element 22 is caused to perform a transition from the OFF state to the ON state.

As a result, the frequency of application of a high voltage (the secondary voltage V2) to the dielectric barrier discharge lamp 10 during a unit time decreases, and therefore the irradiance of light Ry1 decreases. The irradiance of the light Ry1 can be adjusted by appropriately adjusting the OFF holding time Ts. Stated another way, the irradiance of the light Ry1 can be adjusted by making the OFF holding time Ts in a microsecond-scale (1000 µs or less) short lighting period variable.

This also makes it possible to adjust an amount of the light Ry1 emitted from the dielectric barrier discharge lamp 10 without depending on second-scale ON/Off control. Furthermore, this method reduces a frequency of ON/OFF switching of the switching element 22, and therefore a problem of power loss in the switching element 22 is also alleviated.

<3> In the embodiment described above, a case where the switching element 22 is connected between the negative-electrode side terminal of the direct-current power source 21 and the primary winding L1 of the transformer 30 has been described. However, this polarity may be reversed. Stated another way, the switching element 22 may be connected between the positive-electrode side terminal of the direct-current power source 21 and the primary winding L1 of the transformer 30. Here, in a case where the switching element 22 is constituted by a MOSFET, whether the MOSFET is of an n-channel type or a p-channel type is appropriately selected depending on the polarity of the direct-current power source 21 to be connected.

### DESCRIPTION OF REFERENCE SIGNS

- 1: Light source device
- 2, 2a, 2b, 2c: Lighting circuit
- 3, 4: Power line
- 5: Cover
- 6: Main body casing
- 7: Light extraction surface
- 10: Dielectric barrier discharge lamp
- 11, 12: Electrode
- 11a, 12a: Connecting part
- 13: Light-emitting tube
- 21: Direct-current power source
- 22, 22a, 22b, 22c, 22d: Switching element
- 23: Parasitic diode
- 24: Controller
- 25: Smoothing capacitor
- 30: Transformer
- C1: First control
- C2: Second control
- G, G1, G2: Control signal
- I1, I1a, I1b: Primary current
- I2: Secondary current
- L1: Primary winding
- L1a, L1b: Winding
- L2: Secondary winding
- Ry1: Light
- S1: Basic control signal
- Ts: OFF holding time
- V1: Primary voltage
- V2: Secondary voltage
- X1: Starting mode
- X2: Steady-state operation mode
- a1: First terminal
- a2: Second terminal
- b1: First electrode terminal
- b2: Second electrode terminal
- n1: First node
- n2: Second node

## Claims

1. A light source device comprising:
a lighting circuit that includes a direct-current power source, a transformer including a primary winding and a secondary winding, and at least one switching element, the lighting circuit being configured to switch supplying and stopping a current from the direct-current power source to the primary winding of the transformer in accordance with switching of an ON state and an OFF state of the at least one switching element, or change a direction of a current that flows through the primary winding, and generate electromotive force in the secondary winding of the transformer;
a dielectric barrier discharge lamp that is connected to the secondary winding of the transformer; and
a controller that performs ON/OFF control on the at least one switching element, wherein
the controller performs
a starting mode for repeating the ON/OFF control on the at least one switching element at a predetermined frequency to apply a predetermined voltage to the dielectric barrier discharge lamp at a time of starting, and
a steady-state operation mode for alternately performing first control and second control after the dielectric barrier discharge lamp has been started, the first control being performed to repeat the ON/OFF control on the at least one switching element at the predetermined frequency to apply the predetermined voltage to the dielectric barrier discharge lamp, the second control being performed to maintain the at least one switching element in the OFF state during a period of time that is longer than a period of the ON/OFF control on the at least one switching element.

2. The light source device according to claim 1, wherein the controller periodically performs the first control and the second control in the steady-state operation mode.

3. The light source device according to claim 1, wherein the controller performs the steady-state operation mode to vary a number of times of repetition of the ON/OFF control on the at least one switching element in the first control before and after each time of the second control.

4. The light source device according to any one of claims 1 to 3, wherein the controller performs the steady-state operation mode in such a way that a total of an execution time of the first control and an execution time of the second control immediately after the first control is four times or more longer than a period that corresponds to the predetermined frequency, and the execution time of the second control is 100 ms or less.

5. The light source device according to any one of claims 1 to 3, wherein the lighting circuit is a flyback type circuit.

6. The light source device according to any one of claims 1 to 3, wherein the lighting circuit is a push-pull type circuit.

7. The light source device according to any one of claims 1 to 3, wherein the lighting circuit is a full-bridge type circuit.

8. The light source device according to claim 5, wherein the at least one switching element includes a parasitic diode.

9. The light source device according to claim 8, wherein the controller performs, in the first control,
a first step of causing the at least one switching element to perform a transition from the ON state to the OFF state, and
a second step of causing the at least one switching element to perform a transition from the OFF state to the ON state after a lapse of a predetermined OFF holding time from a point in time when a regenerative current flowing through the primary winding has reached a zero value, after the first step.

10. The light source device according to claim 8, wherein the controller performs, in the first control,
a first step of causing the at least one switching element to perform a transition from the ON state to the OFF state, and
a second step of causing the at least one switching element to perform a transition from the OFF state to the ON state after the first step and before a regenerative current flowing through the primary winding reaches a zero value or simultaneously with the regenerative current having reached the zero value.

11. A dielectric barrier discharge lamp lighting circuit configured to light a dielectric barrier discharge lamp, the dielectric barrier discharge lamp lighting circuit comprising:
a direct-current power source;
a transformer that includes a primary winding, and a secondary winding that is connected to the dielectric barrier discharge lamp;
at least one switching element that is connected in series to the direct-current power source and the primary winding of the transformer, and switches supplying and stopping a current from the direct-current power source to the primary winding of the transformer in accordance with switching of an ON state and an OFF state, or changes a direction of a current that flows through the primary winding; and
a controller that performs ON/OFF control on the at least one switching element, wherein
the controller performs
a starting mode for repeating the ON/OFF control on the at least one switching element at a predetermined frequency to apply a predetermined voltage to the dielectric barrier discharge lamp at a time of starting, and
a steady-state operation mode for alternately performing first control and second control after the dielectric barrier discharge lamp has been started, the first control being performed to repeat the ON/OFF control on the at least one switching element at the predetermined frequency to apply the predetermined voltage to the dielectric barrier discharge lamp, the second control being performed to maintain the at least one switching element in the OFF state during a period of time that is longer than a period of the ON/OFF control on the at least one switching element.

12. A dielectric barrier discharge lamp lighting method using a lighting circuit,
the lighting circuit including
a direct-current power source,
a transformer that includes a primary winding, and a secondary winding that is connected to a dielectric barrier discharge lamp, and
at least one switching element,
the lighting circuit being configured to switch supplying and stopping a current from the direct-current power source to the primary winding of the transformer in accordance with switching of an ON state and an OFF state of the at least one switching element, or change a direction of a current to be supplied to the primary winding, and generate electromotive force in the secondary winding of the transformer, and
the dielectric barrier discharge lamp lighting method comprising:
a first step of repeating ON/OFF switching of the at least one switching element at a predetermined frequency to apply a predetermined voltage to the dielectric barrier discharge lamp at a time of starting; and
a second step of alternately performing repeating the ON/OFF switching of the at least one switching element at the predetermined frequency, similarly to the first step, and maintaining the at least one switching element in the OFF state during a period of time that is longer than a period of the ON/OFF switching of the at least one switching element in the first step, in order to maintain a lighting state of the dielectric barrier discharge lamp after execution of the first step.
